# EUROPEAN PATENT APPLICATION

(11) **EP 4 740 828 A1**
(43) Date of publication of application: **13.05.2026**
(21) Application number: 24211324.9
(22) Date of filing: 07.11.2024
(51) Int. Cl.: A61B 1/00, A61B 1/12

(54) **HANDLE ASSEMBLY FOR USE IN A MEDICAL INSTRUMENT AND MEDICAL INSTRUMENT COMPRISING SUCH A HANDLE ASSEMBLY**

(71) Applicant: Olympus Surgical Technologies Europe, 22045 Hamburg (DE)
(72) Inventor: MÜCKNER, Andreas, 22045 Hamburg (DE); JUNGBAUER, Sebastian, 22045 Hamburg (DE)
(74) Representative: Weickmann & Weickmann PartmbB

(57) **Abstract**

The present invention relates to a handle assembly (10) for use in a medical

instrument, in particular an endoscope, comprising a sleave element (12) which delimits an internal space (14) thereof and serves as a structural component, at least one electronic module (16) housed in the internal space (14) of the sleave element (12), and a heat spreader element (20) which is adapted to spread heat produced in the internal space (14) of the sleave element (12) on the outside of the sleave element (12), wherein the heat spreader (20) element also serves as part of an antenna system which is adapted to establish wireless communication between the at least one electronic module (16) and an external communication unit. The invention also relates to a medical instrument, in particular an endoscope, comprising such a handle assembly.

## Description

The present invention relates to a handle assembly for use in a medical instrument, in particular an endoscope, comprising a sleeve element which delimits an internal space thereof and serves as a structural component, at least one electronic module housed in the internal space of the sleeve element, and a heat spreader element which is adapted to spread heat produced in the internal space of the sleeve element on the outside of the sleeve element. Furthermore, the invention relates to a medical instrument, in particular an endoscope, comprising such a handle assembly.

It is known that inside the sleave elements of handle assemblies of medical instruments, heat may be produced which has to be dissipated, for example due to light guides of endoscopes passing through their handle assemblies and in particular fibre couplings being positioned in such handle assemblies which produce a substantial amount of waste heat in small areas such that hot spots inside the handle assembly could occur which might damage its structure and/or impair its functionality.

For these reasons, it is known in the art to position heat spreader elements outside of such sleeve elements which spread the heat produced in certain small areas or hot spots along the extension of the handle in order to prevent localized high temperatures in the corresponding positions as well as large temperature gradients along the respective handles. Such heat spreader elements have oftentimes been implemented by sheet metal elements arranged around the sleeve element delimiting the internal space of the handle assembly such that any temperature gradient originating from certain points or small areas of heat production inside the sleeve element can be distributed and dissipated along the extension of the heat spreader element.

On the other hand, there has been a recent development that certain electronic modules are to be positioned inside the internal space of such handle assemblies, for example sensor units with corresponding processing devices which may detect usage states of the respective medical instruments and provide information representing the usage states to an external device. One such use case may for example be accelerometer data gathered by an electronic module located inside the handle assembly which may be monitored in order to detect unwanted drops or impacts on the instrument.

In such configurations and in particular in use cases in which the corresponding at least one electronic module housed in the internal space of the sleeve element is also to be operated in configurations in which the medical instrument is not connected to a superordinate or external structure by means of communication lines, it can be necessary to establish a wireless communication between the at least one electronic module and an external communication unit by means of an antenna system.

In cases in which the above discussed heat spreader element made of a metal material is provided in a handle assembly also comprising an antenna system, such an internal antenna would be shielded which would drastically reduce its performance. On the other hand, varying the positioning of such antennas in the handle assembly might result in undefined radiation characteristics thereof.

It also has to be kept in mind in this context that the size of an antenna is primarily determined by its transmission wavelength and the lower the frequency, the larger the dimensions of the antenna have to be. The minimum size of the antenna and its mass surface is determined by the lowest frequency in the application and for this reason, devices with integrated antennas cannot arbitrarily be reduced in size without degradation of antenna performance. It also has to be acknowledged that the size of the required ground plane for such an antenna system should also be around the same size as the actual active part thereof which is in conflict with the available space for using a printed circuit board in handle assemblies of typical medical instruments such as endoscopes.

For these reasons, it is the object of the present invention to provide an improved handle assembly which on the one hand allows for spreading heat produced inside its internal space and on the other hand facilitates wireless communication of at least one electronic module housed in its internal space to an external communication unit.

In order to achieve this object and overcome the above discussed shortcomings of the known prior art, the present invention proposes a handle assembly for use in a medical instrument, in particular an endoscope, comprising a sleeve element which delimits an internal space thereof and serves as a structural component, at least one electronic module housed in the internal space of the sleeve element, and a heat spreader element which is adapted to spread heat produced in the internal space of the sleeve element on the outside of the sleeve element, wherein the heat spreader element also serves as part of an antenna system which is adapted to a establish wireless communication between the at least one electronic module and an external communication unit. Thus, by integrating the antenna system and the heat spreader element, the reduced space available in such handle assemblies is used optimally, the required heat spreading capacity can be achieved, and wireless communication with the external communication unit becomes possible in an unobstructed manner.

In particular, a main part of the heat spreader element in the handle assembly according to the present invention may serve as a ground plane of the antenna system and a second part thereof may serve as a radiator part of the antenna system. It shall be pointed out that in particular in scenarios in which the corresponding handle assembly is not mechanically and electrically connected to external devices or superordinate structures, in order to operate the antenna system, a ground plane has to be provided in the handle assembly with sufficient mass. The main part of the heat spreader element preferably being made from metal material is therefore an optimal choice due to both its terminal conductivity and its capability to serve as a ground plane for the antenna system.

In order to achieve the desired wavelength of the antenna system depending on the dimensions of the antenna system, the heat spreader element may at least partially be formed in a helical shape extending around the sleeve element, such that larger antenna lengths are possible. In such embodiments, the heat spreader element may in particular comprise a circular main part serving as the ground plane of the antenna system and a second helical part serving as the radiator part of the antenna system.

In alternative embodiments, the heat spreader element may comprise a circular part serving as the ground plane of the antenna system and a second part serving as the radiator part of the antenna system may be formed as a coil or a dual inverted F-shaped part.

The heat spreader element itself may be formed as a shaped or slotted metal part and be pushed over the sleeve element in order to arrange it at its desired position and/or it may be formed by employing a 3D-MID method.

In order to be able to operate in frequency ranges usually used for wireless communication, the antenna system of the handle assembly according to the present invention may be adapted to transmit in a sub-GHz ISM-band at 868 MHz and/or at 2.400 MHz and/or the λ/4-length of the antenna system may be about 83 mm. However, different frequency ranges may be employed as well, depending for example on the type of medical instrument as well as the preferred communication protocol.

As already briefly discussed above, in certain use cases of handle assemblies according to the present invention, the at least one electronic module may be capable of standalone operation, such that it may comprise a battery and/or it may be adapted to monitor use conditions of the medical instrument by means of at least one sensor unit.

Typically, the sleeve element of the handle assembly according to the present invention may at least be partially made from a plastic material which reduces weight and manufacturing costs, a composite material, a ceramic material or a metal material and/or it may further comprise an outer grip portion, preferably also made from a plastic material, which is positioned outside of the sleeve element and the heat spreader element in a radial direction and adapted to be gripped by the user of the instrument. Thus, in such handle assemblies, the heat spreader element as well as the antenna system are positioned between the sleeve element and the outer grip portion such that there are no metal structures radially outside of the antenna system potentially blocking antenna radiation and any heat produced inside the sleeve element can easily be distributed between the two components in order to prevent any local build-up of heat or hot spots.

As already mentioned above, the present invention also relates to a medical instrument and in particular an endoscope, comprising a handle assembly according to the present invention, which may in particular further comprise a light guide element extending through the sleeve element, for example comprising a fibre coupling assembly positioned at a longitudinal position corresponding to the heat spreader element.

Further features and advantages of the present invention will become even clearer from the following description of embodiments thereof. These show in particular:
- Figure 1:: a schematic cross-section view of a handle assembly according to the present invention; and
- Figures 2a to 2d:: schematic views of different possible configurations of heat spreader elements in the handle assembly of Figure 1.

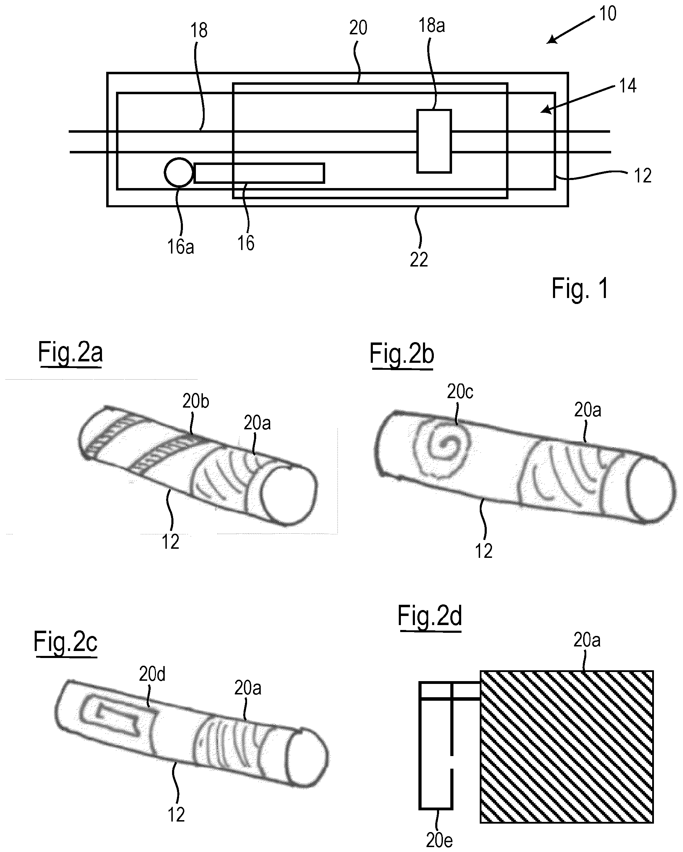

Figure 1 in a schematic cross-section view shows a handle assembly according to the present invention for use in a medical instrument such as an endoscope which is generally denoted by reference number 10. The handle assembly 10 comprises an inner sleeve element 12 which delimits an internal space 14 thereof and serves as a structural component.

Inside the internal space 14 of the sleeve element 12 there is provided an electronic module 16, such as a printed circuit board comprising at least one accelerometer as well as a battery 16a for powering the components of the electronic module 16. Furthermore, a light guide element 18 extends through the internal space 14 of the sleeve element 12, wherein a fibre coupling assembly 18a is provided, by means of which two parts of the light guide element 18 on the right side and on the left side of Figure 1 can be connected and disconnected for an assembly and disassembly of the handle assembly 10 to a superordinate structure. It is expected that in the region of the fibre coupling assembly 18a, substantial waste heat is produced inside the internal space 14 of the sleeve element 12 during operation of the medical instrument which has to be spread or dissipated along the sleeve element 12 in order to prevent a local build-up of heat and excessive temperature gradients in the handle assembly 10.

For this purpose, a heat spreader element 20 which is adapted to spread heat produced in the internal space 14 of the sleeve element 12 is positioned on the outside of the sleeve element 12 in order to spread the heat locally produced inside the sleeve element 12 to a wider area. Said heat spreader element 20 for this purpose is made of a metal material which exhibits high thermal conductivity, such as for example copper. Furthermore, said heat spreader element 20 also serves as part of an antenna system which is adapted to establish wireless communication between the electronic module 16 and an external communication unit. Radially outside of the heat spreader element 20 and the sleave element 12 there is furthermore provided an outer grip portion 22, preferably made of a plastic material, which is adapted to be gripped by a user of the medical instrument.

Four possible embodiments of such heat spreader elements 20 are shown in figures 2a to 2d in schematic views, wherein figures 2a to 2c are isometric views of the respective elements, while figure 2d depicts the corresponding element in an unrolled view.

In the embodiment of figure 2a, the main part 20a of the heat spreader element 20 positioned at a longitudinal position corresponding to the fibre coupling assembly 18a inside the internal space 14 serves as a ground plane of the antenna system 20 and a second part 20b serving as a radiator part of the antenna system 20 is formed in helical shape extending around the sleeve element 12 and in a longitudinal direction thereof.

In alternative embodiments shown in figures 2b and 2c, similar main parts 20a serving as ground planes are provided, however different geometries are used for the respective second parts 20c and 20d, which are in these cases formed as coils with different geometries. Lastly, figure 2d shows an embodiment in which the second part 20e serving as the radiator part of the antenna system is formed as a dual inverted F-shaped part which allows for transmitting in different frequency ranges.

## Claims

1. Handle assembly (10) for use in a medical instrument, in particular an endoscope, comprising:
- a sleave element (12) which delimits an internal space (14) thereof and serves as a structural component;
- at least one electronic module (16) housed in the internal space (14) of the sleave element (12); and
- a heat spreader element (20) which is adapted to spread heat produced in the internal space (14) of the sleave element (12) on the outside of the sleave element (12);
**characterized in that** the heat spreader (20) element also serves as part of an antenna system which is adapted to establish wireless communication between the at least one electronic module (16) and an external communication unit.

2. Handle assembly (10) according to claim 1,
wherein a main part (20a) of the heat spreader element (20) serves as a ground plane of the antenna system and a second part (20b, 20c, 20d, 20e) thereof serves as a radiator part of the antenna system (20).

3. Handle assembly (10) according to any of the preceding claims,
wherein the heat spreader (20) element is at least partially formed in a helical shape extending around the sleave element.

4. Handle assembly (10) according to claims 2 and 3,
wherein the heat spreader element (20) comprises a circular main part (20a) serving as the ground plane of the antenna system (20) and a second helical part (20b) serving as the radiator part of the antenna system (20).

5. Handle assembly (10) according to claim 2,
wherein the heat spreader element (20) comprises a circular main part (20a) serving as the ground plane of the antenna system (20) and a second part (20b, 20c, 20d) serving as the radiator part of the antenna system (20) is formed as a coil or a dual inverted F-shaped part.

6. Handle assembly (10) according to any of the preceding claims,
wherein the heat spreader element (20) is formed as a shaped or slotted metal part and pushed over the sleave element (12).

7. Handle assembly (10) according to any of the preceding claims,
wherein the heat spreader element (20) is formed by employing a 3D-MID method.

8. Handle assembly (10) according to any of the preceding claims,
wherein the antenna system (20) is adapted to transmit in a Sub-GHz ISM band at 868 MHz and/or at 2400 MHz.

9. Handle assembly (10) according to any of the preceding claims,
wherein the at least one electronic module (16) comprises a battery and/or is adapted to monitor use conditions of the medical instrument by means of at least one sensor unit.

10. Handle assembly (10) according to any of the preceding claims,
wherein the sleave element (12) is at least partially made from a plastic material, a composite material, a ceramic material or a metal material.

11. Handle assembly (10) according to any of the preceding claims,
further comprising an outer grip portion (22), preferably made of a plastic material, which is positioned outside of the sleave element (12) and the heat spreader element (20) in a radial direction and adapted to be gripped by a user of the medical instrument.

12. Medical instrument, in particular endoscope, comprising a handle assembly (10) according to any of the preceding claims.

13. Medical instrument according to the preceding claim,
further comprising a light guide element (18) extending through the sleave element, in particular comprising a fibre coupling assembly (18a) positioned at a longitudinal position corresponding to the heat spreader element (20).
